# EUROPEAN PATENT SPECIFICATION

(11) **EP 3 133 953 B1**
(45) Date of publication and mention of the grant of the patent: **12.02.2020**
(21) Application number: 15718219.7
(22) Date of filing: 23.04.2015
(51) Int. Cl.: A45D 20/12, A45D 24/00

(54) **HAIRDRESSING APPARATUS**
FRISIERVORRICHTUNG
APPAREIL DE COIFFURE

(30) Priority: 23.04.2014 NO 20140529
(43) Date of publication of application: 01.03.2017
(73) Proprietor: Kan Holding AS, 0251 Oslo (NO)
(72) Inventor: NILSEN, Kåre A., 1362 Hosle (NO)
(74) Representative: Onsagers AS
(86) International application number: PCT/EP2015/058811
(87) International publication number: WO 2015/162209

(56) References cited:
- EP-A1- 0 813 827
- DE-A1- 2 508 951
- DE-A1- 2 529 809
- DE-U1- 8 028 151
- FR-A1- 2 725 346
- US-A1- 2012 123 305

## Description

### Introduction

The present invention concerns a hairdressing apparatus. More specifically, a hairdressing apparatus is disclosed, for concurrent combing and drying of hair.

### Background

Subsequent to a hair washing, or other wetting of hair, the strands of hair has a tendency to stabilize with a certain elastic rigidity in the form and position they assume during drying. During hairdressing, when air is supplied, and, specifically when hot air is supplied, e.g., with a so called blow-dryer, it is thus important to get the strands of hair into a desired form and position during the drying procedure. More specifically, in the dressing of relatively short hairstyles, such as a typical male hairdo or a short hair female hairdo, it is important, during the drying, that the hairs do not lay flat against the scalp, but rather are raised in an arc in the direction of dressing the hair, such that it is possible to create relatively tall hair styles having an attractively full appearance. Usually, this is achieved when the hair dresser leads the blow-dryer slowly across the head with one hand, and, at the same time, the other hand lifts up the hair in front of the air flow using a comb, which, however, is something that requires a lot of practice and a steady hand to produce a desired result.

However, it is already known to perform combing and supplying of hot air in one operation using some sort of brush having hollow teeth attached to a hot air distributing chamber which is designed as a handle, and wherein the air is blown towards the hair during combing.

DE 2 137 407 discloses a known embodiment in which the teeth are designed as hollow tubes through which the air flows from a distributing chamber towards openings close to the tips of the teeth, and, wherein the distribution chamber between the teeth have additional openings, from which the air is blown directly down toward the hair and scalp. The side openings partly face one side, and partly the other side, relative the longitudinal axis of the brush. The result is that the air is blown partly directly down toward the hair, and partly with an inclination toward the scalp in different directions. The sideways directed outlet openings in the teeth will produce a direct drying action at the root of the hair strands. The component of the air streams directed down toward the scalp will however cause the hair to lay down flat, such that stabilization by direct drying action will be performed on strands of hair which lay flat. And, because the air must deflect along the scalp, the drying action occurs along the top of the hair strands because of the specific holes in the distributor, and the blowing action occurs straight down, which further contributes to flatten the hair strands. Said stabilization action is thus irregular because of the different orientation of the air streams from the teeth. Consequently, with such apparatuses, it is difficult to achieve results as good as those achieved by an experienced hair dresser concurrently using a comb and a blow-dryer. Applicant's own patent NO 135966 was directed to solve this problem by making it possible, in a convenient way, to achieve good results in an operation using only one hand. The apparatus disclosed in said patent has teeth attached to a hollow grip which works as a hot air distribution chamber, for concurrent combing and drying, and the teeth are constituted by hollow tubes which are joined to the distribution chamber. The apparatus is characterized in that the end parts of the teeth are all curved in the same direction, and have outlet openings which are directed backwards with an inclination.

This apparatus provides a substantial improvement. However, it has disadvantages which limit the combing effect, because the tips of the teeth are curved and they must have a certain width to allow air to flow out from the tip of a tooth. Another disadvantage when hot air is supplied is that the scalp quickly heats up, and thus discomfort results.

The document DE2529809A1 discloses a hairdressing apparatus with teeth having openings formed as channels.

The document FR2725346A1 discloses a hairdressing apparatus comprising several teeth with several openings thereon.

The present invention solves the above mentioned problems by a novel and inventive design of the teeth in a hairdressing apparatus, in which good combing effect is achieved, and at the same time, the bottom part of the hair strands is quickly dried by supplying hot air, without the scalp getting heated up. Thus, the hairdressing apparatus may be used over extended periods of time, without discomfort.

Because of improved combing effect with good parting of hair strands, a better airing effect will be achieved, such that the drying and dressing process of the hair is performed faster than in prior art processes.

The document DE2508951 A1 discloses a hairdressing apparatus with teeth comprising inclined openings.

### Short description of the invention

The present invention discloses a hairdressing apparatus for dressing hair. The hairdressing apparatus comprises a plurality of hollow teeth which are attached to a hollow grip, which grip function as an air distribution chamber for directing air into the teeth when air is supplied to the hollow grip.

The invention is characterized by claim 1.

Further features of the invention are defined in the set of claims below.

### Detailed description of the invention

In the following, a hairdressing apparatus will be described in detail, with reference to the figures, wherein the figures present an embodiment not forming part of the present invention, but whose description helps to understand the invention.
Figure 1 is a 3D representation of a partly cut hairdressing apparatus;
Figure 2 is a section of a cut through hairdressing apparatus, and
Figure 3 is a section A-A in figure 2 of a detail of a tooth.

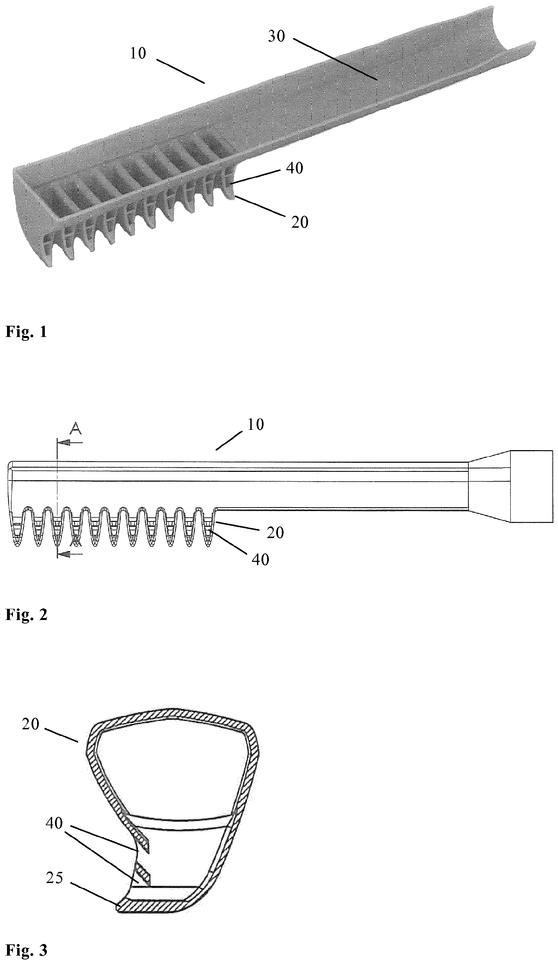

Figure 1 is a 3D representation of a hairdressing apparatus 10 for concurrent combing and drying of hair. In the figure, a hollow grip 30, which function as a air distribution chamber, is shown cut through to expose the inner constructional features with chambers ensuring an air connection between grip 30 and teeth 20. By supplying air at the end of the grip 30 farthest away from the teeth 20, air will be distributed to each tooth 20.

The grip 30 may be formed for adaption to the outlet of a hot air source, e.g., a blow-dryer, a hose with a nipple etc., from where the hot air is blown into the end of the grip 30.

The hairdressing apparatus 10 may in one embodiment also be an independent unit which by itself generates a stream of air, such as is known from present blow dryers. Then, air will be supplied to the hollow grip 30 from internally self generating means.

Air supplied to each tooth 20 will be pressed against the hair, and upward from the scalp, because the teeth 20 have at least one outlet opening 40 above its tips 25 which are situated closest to the scalp, and wherein at least one outlet opening 40 is formed as a channel in the combing direction and having an inclination in a direction upward from the tips 25 of the teeth 20. By the outlet opening 40 being placed above a tip 25, it is meant that the outlet opening 40 is not placed on the tip 25 itself, but in an area a distance above this tip, e.g., a distance from 1-5 mm, as indicated in the figures.

There are several advantages to this embodiment, such as improved combing and better shaping of hair when supplying air, preferably hot air. The constructional features of the hairdressing apparatus 10 makes it possible to work in the same area without the danger of the scalp being heated, such as is the case with the prior art solutions.

When using the hairdressing apparatus 10, and the brush is pulled through wet hair, hot air will be supplied in the combing direction and quickly dry shape the bottom part of the hair strands, and at the same time, avoiding the danger of heating up the scalp itself. The hairdressing apparatus 10 will also contribute to raise the hair strands such that they are stabilized in a desired position. The result is that one achieves a dried hair with a full look, and wherein one is free to give shape to a desired hairdo and decide the direction of the hair when in the dried condition.

Figure 1 shows a set of teeth 20 in one row. This is an example of one embodiment.

It is also possible to have teeth 20 in two or more rows. If there is more than one row, each row may for instance be somehow shifted in relation to each other.

Figure 2 shows a section through a hairdressing apparatus 10. Here, an example of a hairdressing apparatus 10 having eleven teeth 20 is shown.

Figure 3 is a section A-A from figure 2, and shows constructional details for a tooth 20. The figure shows an embodiment in which the teeth 20 have a somewhat curved shape. In a preferred embodiment, the teeth 20 are substantially straight to achieve optimal combing effect.

Each tooth 20 has as mentioned at least one outlet opening 40, through which air may pass, such that the hair is dried. In an embodiment as shown in figure 3, there are two outlet openings 40 having channels which are arranged inclined in a direction upwards from the tip 25 of the tooth 20. The two channels are thus angled in the same direction. According to the invention, but not shown in the figures, the outlet openings 40 are angled in different directions, such that air streams passing through them may be directed to the desired area of the hair.

To achieve narrow teeth 20, according to the invention, the outlet openings 40 are arranged above each other, i.e., they are placed vertically on top of each other in each tooth 20. The outlet opening 40 may in another embodiment also be arranged horizontally adjacent each other. In a further embodiment, the outlet openings 40 may for instance be arranged vertically on top of each other, and horizontally adjacent to each other in alternate teeth 20.

The outlet opening 40 may have a different cross section, which is also evident from figures 1 and 3, in which it may be seen that the tooth 20 is tapered towards its tip 25.

The width of the teeth may typically be between 1 and 5 mm. There will be a consideration between desired combing effect and blow-drying effect. Narrow teeth 20 produce a better combing effect, while broad teeth 20 produce a better blow-drying effect.

As can be seen, any combination of the number of outlet openings 40, arrangement and cross section for these as well as the width of the teeth 20, is possible within the scope of the present invention.

The hairdressing apparatus comprises in one embodiment a light source which is placed in or at each tooth 20, and which is directed towards the tips 25 of the teeth 20 for generating energy for stimulating the scalp, and at the same time provide a heating effect which additionally dries and shapes hair.

Said light source may in an embodiment be placed between each tooth 20, i.e., in the area of the hairdressing apparatus 10 from which each tooth 20 originate. The light source may also be placed in each tooth on the tip, or in an area close to the tip 25 of a tooth 20. An embodiment of the hairdressing apparatus 10, wherein the light sources are placed between each tooth 20 as well as in each tooth 20, is also possible.

The light sources are preferably laser light having high energy, but may also be IR light or a combination of these.

Following performance of a combined combing and drying operation, the final hairdressing work may be carried out, without risking the strands of hair losing their resilience, and desirably during continued use of the hairdressing apparatus 10 as a common hair brush. Using the combined combing, drying and airing action, the present hairdressing apparatus 10 achieves a stabilization of the strands of hair in a desired shape and position irrespective of the prior positioning of the hair, that is, if the hair for instance is laying in, against, or is differently oriented in relation to the direction of combing.

The present invention provides several advantages than what is previously known from corresponding devices. The following advantages may be mentioned: improved combing and airing effect, heating of the scalp is avoided, teeth 20 may be formed narrow, provide better effectivity and time saving.

All these advantageous features makes the present invention contribute to a hairdressing apparatus 10 which is both effective concerning drying and shaping of hair, as well as cost effective.

## Claims

1. Hairdressing apparatus (10) for shaping hair, comprising a plurality of hollow teeth (20) attached to a hollow grip (30), said grip (30) acting like an air distribution chamber for supplying air to the teeth (20) when air is supplied to the hollow grip (30), wherein each tooth (20) has at least two outlet openings (40) on only one side of the tooth facing the combing direction, wherein the at least two outlet openings (40) are placed vertically on top of each other above the tip (25) of the tooth, and wherein the at least two outlet openings (40) are formed as channels each having an inclination in a direction upwards from the tip (25) of the teeth (20), such that air will be pressed against the hair and up from the scalp, and where the at least two outlet openings (40) are inclined in different directions.

2. The hairdressing apparatus (10) according to claim 1, **characterized in that** the teeth (20) are substantially straight.

3. The hairdressing apparatus (10) according to any one of claims 1 to 2, **characterized in that** the outlet openings (40) are additionally provided horizontally next to each other.

4. The hairdressing apparatus (10) according to any one of claims 1 to 3, **characterized in that** the outlet openings (40) have different cross sections.

5. The hairdressing apparatus (10) according to any one of claims 1 to 4, **characterized in that** the width of the teeth (20) is 1-5 mm.

6. The hairdressing apparatus (10) according to claim 5, **characterized in that** the teeth (20) have the same width.

7. The hairdressing apparatus (10) according to claim 5, **characterized in that** the teeth (20) have different widths.

## Patentansprüche

1. Frisiervorrichtung (10) zum Formen von Haar, umfassend eine Vielzahl von hohlen Zähnen (20), die an einem hohlen Griff (30) befestigt sind, wobei der Griff (30) wie eine Luftverteilungskammer zum Zuführen von Luft zu den Zähnen (20) wirkt, wenn Luft in den hohlen Griff (30) geleitet wird, wobei
jeder Zahn (20) mindestens zwei Auslassöffnungen (40) auf nur einer Seite des Zahns aufweist, die zu der Kämmrichtung ausgerichtet ist, wobei die mindestens zwei Auslassöffnungen (40) vertikal übereinander über der Spitze (25) des Zahns angeordnet sind, und wobei die mindestens zwei Auslassöffnungen (40) als Kanäle ausgebildet sind, die jeweils eine Neigung in einer Richtung von der Spitze (25) des Zahns (20) nach oben aufweisen, so dass Luft gegen das Haar und nach oben von der Kopfhaut gedrückt wird, und wobei die mindestens zwei Auslassöffnungen (40) in verschiedene Richtungen geneigt sind.

2. Frisiervorrichtung (10) nach Anspruch 1,
**dadurch gekennzeichnet, dass** die Zähne (20) im Wesentlichen gerade sind.

3. Frisiervorrichtung (10) nach einem der Ansprüche 1 bis 2,
**dadurch gekennzeichnet, dass** die Auslassöffnungen (40) zusätzlich horizontal nebeneinander vorgesehen sind.

4. Frisiervorrichtung (10) nach einem der Ansprüche 1 bis 3,
**dadurch gekennzeichnet, dass** die Auslassöffnungen (40) unterschiedliche Querschnitte aufweisen.

5. Frisiervorrichtung (10) nach einem der Ansprüche 1 bis 4,
**dadurch gekennzeichnet, dass** die Breite der Zähne (20) 1-5 mm beträgt.

6. Frisiervorrichtung (10) nach Anspruch 5,
**dadurch gekennzeichnet, dass** die Zähne (20) dieselbe Breite aufweisen.

7. Frisiervorrichtung (10) nach Anspruch 5,
**dadurch gekennzeichnet, dass** die Zähne (20) unterschiedliche Breiten aufweisen.

## Revendications

1. Appareil de coiffure (10) pour mettre des cheveux en forme, comprenant une pluralité de dents creuses (20) fixées sur une poignée creuse (30), ladite poignée (30) servant de chambre de distribution d'air pour fournir l'air aux dents (20) lorsque l'air est amené à la poignée creuse (30), dans lequel :
chaque dent (20) a au moins deux ouvertures de sortie (40) uniquement d'un côté de la dent faisant face à la direction de coiffage, dans lequel les au moins deux ouvertures de sortie (40) sont placées verticalement l'une sur l'autre au-dessus de la pointe (25) de la dent, et dans lequel les au moins deux ouvertures de sortie (40) sont formées comme des canaux, ayant chacune une inclinaison dans une direction ascendante à partir de la pointe (25) des dents (20), de sorte que l'air peut être comprimé contre le cheveu et remonté à partir du crane, et dans lequel les au moins deux ouvertures de sortie (40) sont inclinées dans différentes directions.

2. Appareil de coiffure (10) selon la revendication 1, **caractérisé en ce que** les dents (20) sont sensiblement droites.

3. Appareil de coiffure (10) selon l'une quelconque des revendications 1 à 2, **caractérisé en ce que** les ouvertures de sortie (40) sont prévues, de manière supplémentaire, horizontalement l'une à côté de l'autre.

4. Appareil de coiffure (10) selon l'une quelconque des revendications 1 à 3, **caractérisé en ce que** les ouvertures de sortie (40) ont des sections transversales différentes.

5. Appareil de coiffure (10) selon l'une quelconque des revendications 1 à 4, **caractérisé en ce que** la largeur des dents (20) est de 1 à 5 mm.

6. Appareil de coiffure (10) selon la revendication 5, **caractérisé en ce que** les dents (20) ont la même largeur.

7. Appareil de coiffure (10) selon la revendication 5, **caractérisé en ce que** les dents (20) ont des largeurs différentes.
